# EUROPEAN PATENT APPLICATION

(11) **EP 2 613 276 A1**
(43) Date of publication of application: **10.07.2013**
(21) Application number: 12425001.0
(22) Date of filing: 04.01.2012
(51) Int. Cl.: G06F 19/00

(54) **Method and apparatus for neuromotor rehabilitation using interactive setting systems**

(71) Applicant: Ceruti, Gabriele, 24010 Treviglio (BG) (IT); Orlandi, Thomas, 24040 Arcene (BG) (IT); Manzini, Veronica, 24100 Bottanucco (BG) (IT); Rispoli, Jessica, 24050 Lurano (BG) (IT)
(72) Inventor: Ceruti, Gabriele, 24010 Treviglio (BG) (IT); Orlandi, Thomas, 24040 Arcene (BG) (IT); Manzini, Veronica, 24100 Bottanuco (BG) (IT); RIspoli, Jessica, 24050 Lurano (BG) (IT)
(74) Representative: Bruni, Giovanni

(57) **Abstract**

The invention provides a method and an apparatus for neuromotor rehabilitation by using interactive setting systems which allow the subject or patient to carry out rehabilitative training, and to collect functional parameters concerning the neuromotor aspect. Said method offers in only one apparatus a play means, a rehabilitative equipment and a device for collecting and analyzing specific medical parameters. The method can be used according to two modes: the assisted and independent one. The subject is represented in a virtual setting in which he is asked to carry out precise activities in order to reach a target aim and, at the same time, to use or summon up motor pattern studied by rehabilitation therapists; during the activity the equipment records a very significant quantity of previously selected data which are classified, processed, compared and analyzed by means of a dedicated data management software.

## Description

The present invention relates to a method and apparatus for neuromotor rehabilitation using interactive setting systems allowing the subject or patient to make rehabilitation training. The invention provides the whole process and includes the collection of subject functional parameters concerning the neuromotor aspect, the subject functional evaluation, the rehabilitative program planning up to the real sessions. Specifically, the present invention relates to a method and apparatus to set up rehabilitative sessions by means of virtual settings and play activities, but still based on scientific evidence (EBM - Evidence Based Medicine). In fact, the subject suffering from motor skills disorder is asked to reach specific aims during an activity in virtual settings, while at the same the equipment collects significant data in real time to evaluate the neuromotor and osteoarticular deficit.

Such settings are original, exclusive, validated and convenient since they are studied by specialized staff, and defined and created as well for each patient and disorder, so that specific muscular areas are summoned up and specific motor units and specific osteoarticular districts are activated. The subject suffering from a particular motor skills disorder or the supervisor can choose, among a series thereof, the setting "pack" with which to interact to evaluate/rehabilitate the specific motor skills disorder.

These settings are made up and/or validated by specialized and skilled professional teams who can satisfy the movement needs of a specific area.

In the state of the art, and above all recently, a new joypad concept, which transforms actually the body in a system of virtual control, has had a huge improvement in the field of the interactive games. This fact is proved by the strong marketing and diffusion of new consoles which require no more only a digital performance, intended as the use of a joystick and so of only some fingers of the hand, but a more global body performance able to reproduce its movements and to allow the interaction in a virtual setting. This technological evolution has been followed by many fields. Unfortunately, in medicine and above all in the rehabilitation field such gap has not been totally plugged. What is needed is the ability to understand the evolutions and changes to get a global view of the reality and to be able to choose the best solutions in order to provide suitable and efficient rehabilitative "settings". More recent studies in the neuroscience field have led to a progressive evolution of the rehabilitative offer, which is no more limited to a strictly mechanistic view but which is aimed at the subject in his entirety. This more holistic view is well matched with the virtual settings created for this new kind of interactive game; during the play activity, in fact, the performances required do not concern only an action, but an action integrated in a target context (the whole body in involved to reach an aim). This aspect is well highlighted by recent studies, in which the cerebral activity is monitored and recorded during the performance of a functional activity. The rehabilitative world is still considered empirically ("I do and see after if it works"). The method and apparatus provided are adapted to the de facto standard in which the concept is based on the scientific evidence ("I record the approach by means of evidence"). Currently, no such equipments are provided in the market or however they are not theoretically available. There are available equipments which record the walking analysis and which are able to record and divide the mono and bipedal charge.

There exist also consoles for play or recreational activities, but which have nothing to do with medical aims. The present invention provides a method which uses only one apparatus able to represent, by a play means, a rehabilitative apparatus and an equipment to collect and analyze specific medical parameters.

These and other advantages will be better highlighted in the following description of the invention, which refers specifically to figures 1 - 7, in which a preferred, not absolutely limiting, embodiment of the present invention is shown. In particular:
Fig. 1 shows a diagram representing the components involved in the method;
Fig. 2 shows a block diagram representing as a way of example the performance of the method for carrying out a rehabilitative session;
Fig. 3 shows a block diagram representing as a way of example the method for loading or adjusting the data primary pattern;
Fig. 4 shows a block diagram representing as a way of example the method for the setting performance and the data acquisition;
Fig. 5 shows a block diagram representing as a way of example the method for managing the acquired data;
Fig. 6 shows a block diagram representing as a way of example the method for analyzing the filed data;
Fig. 7 shows a block diagram representing as a way of example the method for choosing and defining the corrective actions.

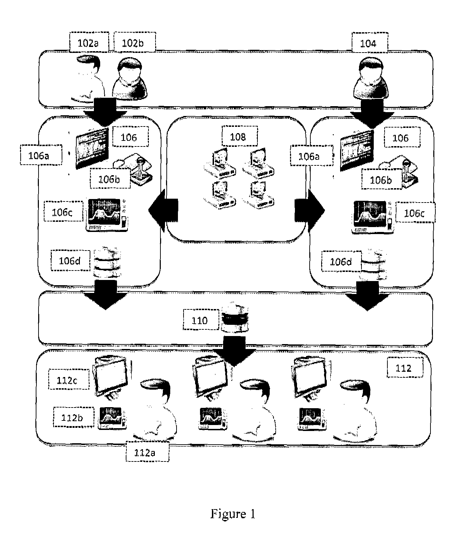

Particularly referring to said figures, the method abject of the present invention can be followed according to two modes (figure 1); the assisted and independent one. In the "assisted mode", two fundamental parties are the subject (patient) 102b and the supervisor 102a. In the "independent mode" there is only one party involved and corresponds to the subject who has to carry out the action 104. The indicated subjects interact actively with the apparatus 106. The apparatus 106 is made up of a console 106a which is the interface with respect to the output devices as the video and/or audio ones, a series of sensors 106b detecting and measuring each movement carried out during the action by the subject, an algorithm 106c for screening, filing and analyzing the data collected by the sensors 106c and a local data base 106d in which the thus screened and analyzed data are filed. The elements shown in the box 108 represent all the settings ideated ad hoc by specialized rehabilitation therapists to activate specific movements. In case the subject allows the data to be remotely input, the data are sent to the remote database 110. The data collected in the central database 110 can be analyzed 112 by supervisors and/or certification authorities 112a. In turn, the subjects use systems 112c connected to the remote database with specific data aggregation algorithms 112b to define and present data concerning the whole population.

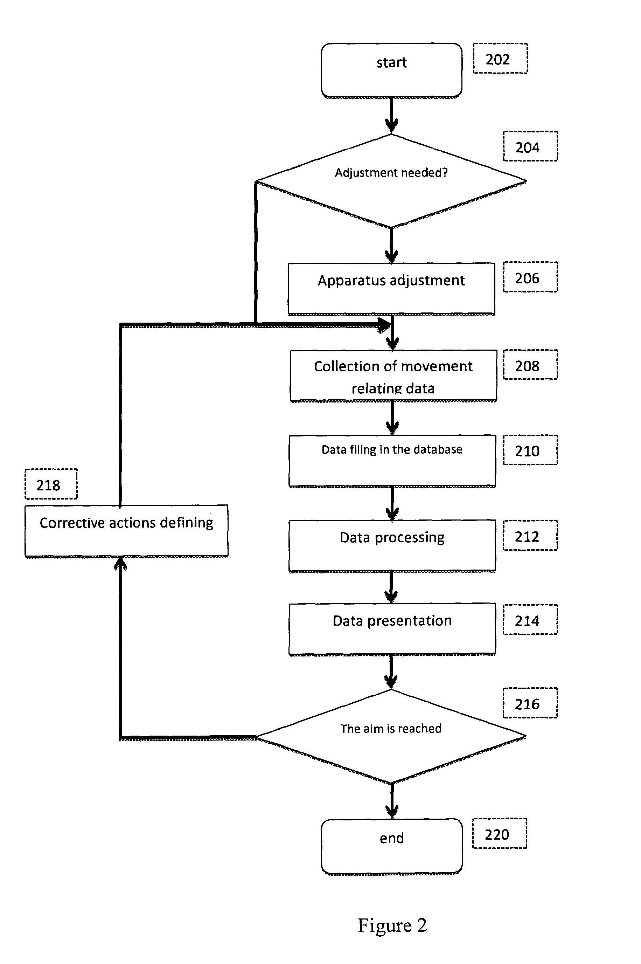

The method 200 to carry out a rehabilitative session (figure 2) begins at step 202 and goes to step 204 where it is asked if it is needed to carry out the adjustment of the equipment or the interactive setting. In the affirmative case, the method goes to step 206 where specific movements are asked to the subject in order to collect the measures considered as primary pattern. The method goes to step 208 where the subject is asked to complete the created ad hoc actions which require specific movement sequences. In the method it is provided, upon approval, the function of uploading the collected data in order to share the improvements with a supervisor, both locally and remotely, and to edit medical statistics. The method goes to step 212 where the data collected at step 208 are analyzed by an algorithm. The algorithm, after taking in input the collected values, produces evaluation indicators in output. Besides involving the new sample, the data processing can consider also the preceding ones in order to provide a time analysis (gap analysis). At step 214, the method provides to the same subject or supervisor an intelligible representation of the calculated indicators in order to allow the self-evaluation or evaluations of the improvement/regression made. At step 216, after the analysis at the preceding step, it is asked if the result allowed to reach the aim or not. In the negative case, the method goes to step 218 where the supervisor or the same setting modifies the difficulties of the action to be carried out. The method 200 ends at step 220.

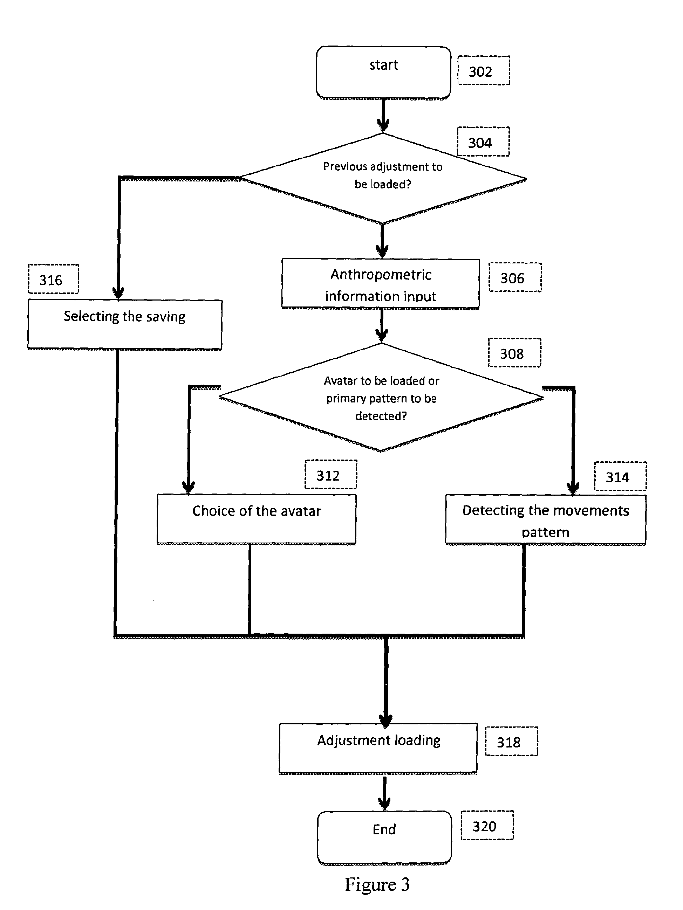

The method 300 for loading or adjusting the data primary pattern (figure 3) begins at step 302 and goes to step 304 where it is asked if it is needed to load a previous adjustment or if it is needed to proceed to the creation of a new data primary pattern with a new adjustment. In case it is required a new data primary pattern, the method goes to step 306 where anthropometric and personal information are asked (as for example weight, height, gender, etc...). After the information input, the method 300 goes to step 308 where it is asked if it is needed to load a pre-configured avatar or if it is intended to carry out some basic movements to create a personalized avatar. In the case it is intended to use a pre-set avatar the method goes to step 312 where it is asked to choose the avatar which better corresponds to the subject who is using the apparatus. The avatar is created by means of filing and standardization of the respective movement data of various "normal" subjects with heterogeneous physical-somatic features. In particular cases, as for example the rehabilitation or the pathologies' treatment, the primary pattern can be created using the movement of the limb opposite to the side suffering from the motor skills disorder. In this case, the method 300 goes to the step 314 where the subject is asked to carry out specific movements with the healthy limb to define the primary pattern. At step 304, in case it is chosen to load a previous adjustment, the method goes to step 316 where the subject is asked to choose the desired adjustment. After defining the primary data set the method goes to step 318 where the filed or recorded data are activated according to the setting chosen. The method 300 ends at step 320.

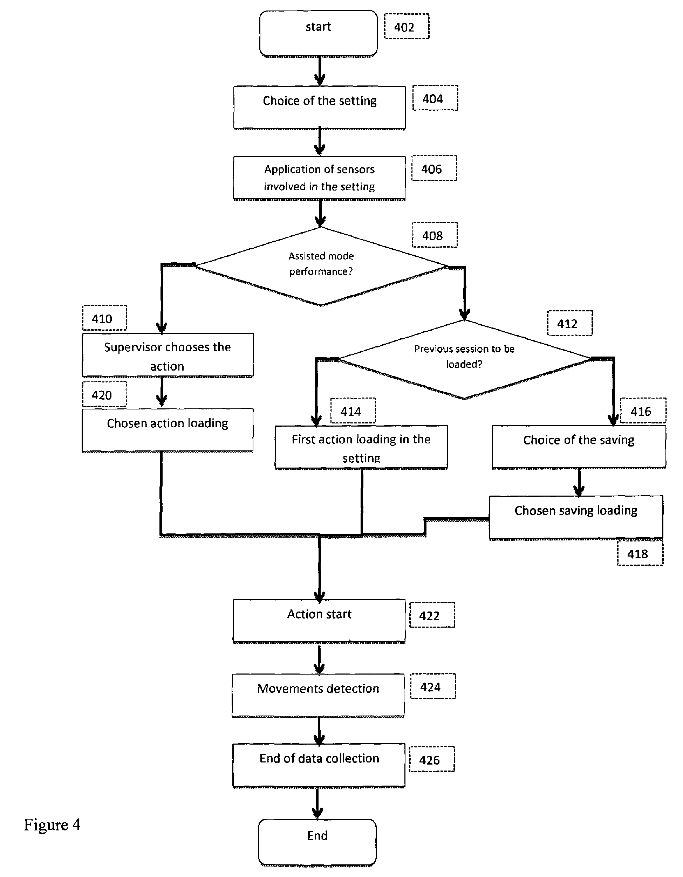

The method 400 for setting performance and data acquisition (figure 4) begins at step 402 and goes to step 404 where it is chosen the setting where the actions are carried out and the data related to the requested movements are acquired. The setting choice is functional to the aim pursued and can be created ad hoc to stimulate the interaction level with the subject. For subjects with sport preferences it is possible to choose theme settings focused on the sport, for subjects with "adventure" preferences it is possible to choose theme settings focused on plots created ad hoc. At step 406, the subject or supervisor is asked to apply the sensors according to the specific requirements of the setting chosen at step 404. The sensors can be applied directly on the body of the subject, or they can be devices (platforms, mats, gloves, socks, etc...), with which the subject interacts. The method 400 goes to 408 where it is asked the mode with which the setting is interacted with; it is defined "assisted mode" when the subject interacts with the setting helped by the supervisor (as for example in sessions in health centres, hospitals, gyms, etc...) while, it is defined "independent mode" when the subject interacts independently with the setting and chooses the actions. In case it is activated the "assisted mode", the method goes to step 410 where the supervisor chooses the action to be carried out by the subject according to performance data provided by the apparatus and according to his own medical know-how. From step 410 the method goes to step 420 where it is loaded the action chosen by the supervisor. In the case, the equipment is on "independent mode", the method goes to step 412 where the subject is asked if he intends to begin from the first action or if he desires to start again from the last successfully completed action. If the subject intends to begin a new action, the method 400 goes to step 414 where the data of the first action are loaded. In case the subject desires to maintain the improvement made and continue from the last successfully completed action, the method goes to step 416 where it is asked to select the saving from which it has to be loaded. From step 416, the method goes to step 418 where the action indicated by the selected saving is loaded. At step 422, the method 400 starts the action loaded in the previous step. While the action is carried out, the method detects the movements (step 424). At the end of the action, the method goes to step 426 where the acquired data are collected and the perceived pain level evaluated for the next filing step. The method ends at step 428.

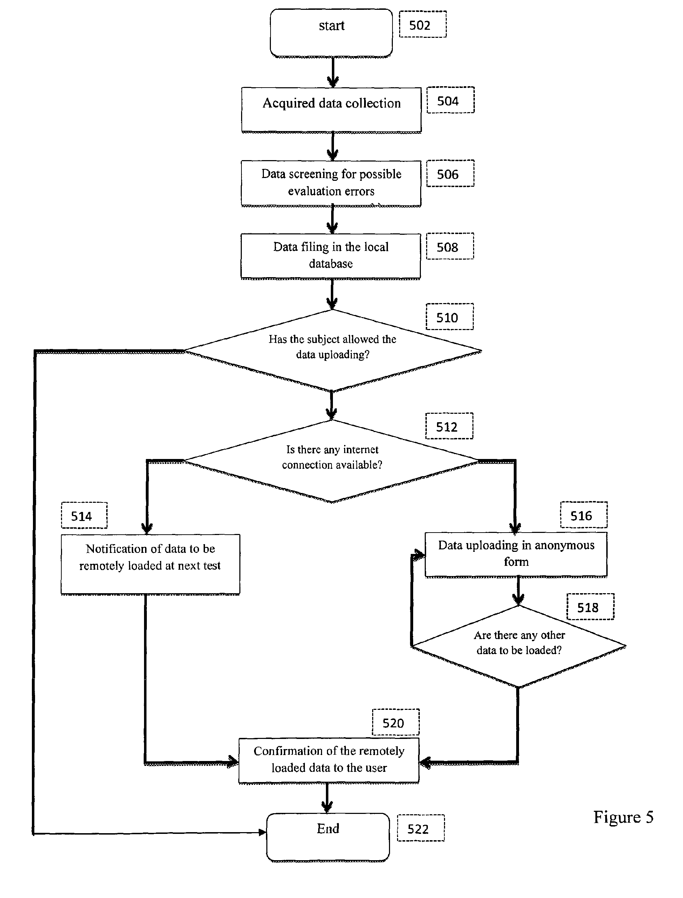

The method 500 for managing the acquired data (figure 5) begins at step 502 and goes to step 504 where the data obtained during the action performance, which was chosen and carried out at previous step (method 400), are collected. The method goes to step 506 where the collected data are screened in order to evaluate the movements carried out without the influence of acquisition errors of the equipment. At step 508, the method proceeds to the data filing in the local data base. The datum indicating the movement is represented by a structure created ad hoc to contain the information concerning the movement in raw form. For the final evaluation it is important to have all the detected evaluation parameters since the specific indicators are obtained by aggregating the data in raw form. At step 506, the method files the data screened at step 504 in a local data base. The data base has to be able to go back to and describe the single actions associated to the respective subjects. Such a description allows to carry out analysis based on time scale and so to draw an improvement graph. At step 510 the method verifies if the subject has allowed or not the loading of the data relating to the single setting/action in a national/international data base. In case the subject did not allow the loading, the method goes to step 522. In case the subject allowed the use and thus the remote loading, the method 500 proceeds to the control of the internet connection. If the internet connection is available and on, the method goes to step 516 where it proceeds to the remote loading of the thus filed data. The remote structure of the data base corresponds to the one locally available in each setting. After carrying out the loading of the just completed action data, the method verifies that there are no more data to be loaded. In affirmative case, the method goes from step 516 to step 518, where it controls that no more movements set, previously filed but not yet loaded, are present. In case no internet connection is available, the method 500 goes to step 514 where it tags "to be loaded" the recorded data. As previously described, these data are loaded as soon as the control of the connection results positive. The steps 518 and 514 proceed to step 520 where the real loading of the data in the remote data base is confirmed. From step 520, the method goes to step 522. The method ends at step 522.

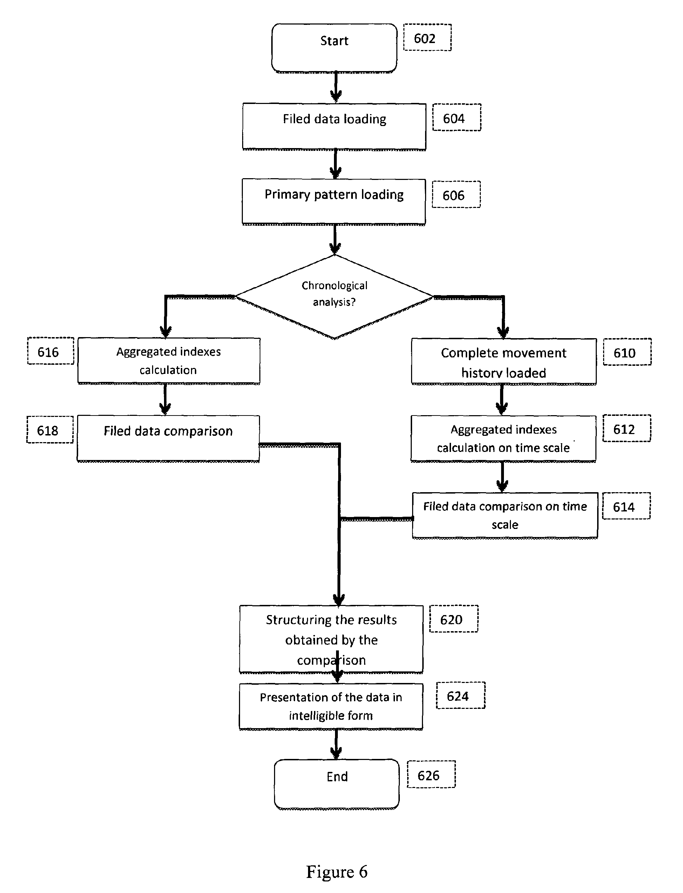

The method 600 for the analysis of the filed data (figure 6) begins at step 602 and goes to step 604 with the loading of the data acquired after the last action is completed. After the loading of these data, the method goes to step 606 with the loading of the data primary pattern relative to the same setting. At step 608, the method asks if the analysis has to be carried out on the single action or on time scale. In case the analysis has to be carried out on the single action, the method goes to step 616 where the aggregated indexes, i.e. which are not available as raw data during the data acquisition, are calculated. After the index calculation at step 616, the method goes to step 618 where the data are actually compared with the raw pattern. At step 608, in case it is intended to carry out an analysis on time scale, the method goes to step 610 where the data relative to each action carried out by the subject for that precise setting are loaded. At step 612 the method calculates the indexes generated by the aggregation of the data loaded at the previous step. At step 614, the method carries out the comparison, on time scale, of the data calculated at the previous step.

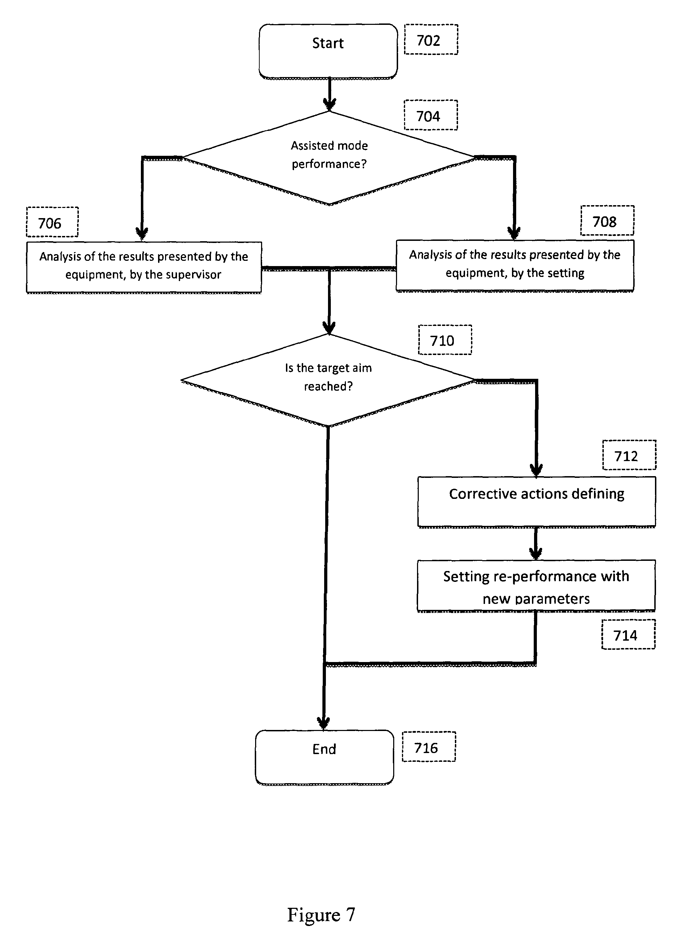

The steps 618 and 620 proceed to step 622 where the comparison results are collected and structured for the next presentation. At step 624, according to the performance mode (assisted or independent one), the method presents the results in intelligible form in order to reach conclusions and make the following corrections. The method ends at step 622. The method 700 for choosing and defining the corrective actions (figure 7) begins at step 702 and goes to step 704 where the method verifies if it is working in "assisted mode" or in "independent mode". In case it is working in "assisted mode", the method goes to step 706 where the supervisor evaluates the results presented and calculated by the previous diagram. In case the method is working in "independent mode", it goes to step 708 where the evaluations are made by the same setting. The method goes from step 706 and 708 to step 710 where it is evaluated if the aim pursued by the action carried out has been reached or not. In the negative case, the method goes to step 712 where the corrective actions to be applied next are defined. After defining the corrective actions at step 712, the method goes to step 714 where the method carries out again the action just completed, with the new set up parameters. From steps 710 and 714, the method goes to step 716. The method ends at step 716.

The equipment is made up of the following hardware: a motion controller, a posturometric platform, a series of sensors, and a dedicated software which manages both the reproduction of the virtual settings and the processing of the collected specified data and their presentation. The stabilometric platform is able to measure exactly the barycentre, the mass and the body weight of the user. It uses the Bluetooth technology and contains pressure sensors used for the measurements. The platform sends data to a specific software which processes them; this allows to display both numerically and graphically the information concerning barycentre, pressure of the lower limbs, movement speed in real time. In addition to the data displaying and analyzing, other two processes are carried out at the same time: the data chronologizing in the database for next processing and interfacing with the virtual setting to communicate that the subject is performing. The motion controller is a technology substantially based on a camera able to measure the distance between objects and surfaces on the basis of a scanning carried out by means of infrared radiation and peculiar for its possibility to interface with the user without any physical contact. This guarantees that even subjects with significant movement dysfunctions are able to carry out movements sequences.

The distance measurement occurs thanks to the synergy between the infrared projector generating a grid of points invisible to the human eye and a sensor which captures the reflections of such infrared radiation and evaluates the way in which the information come back to it. The motion controller communicates to the software the detections inside its own visual spectrum. In order that the detections are reliable it is needed a calibration. Once the user has been recognized, twenty-two points of the body are detected with three coordinates for each point (3D), which are processed similarly to what above described concerning the platform; real time display of the movements and their analysis, communication with the virtual setting to guarantee the interaction with the user and data chronology. The detectable points are: shoulders, elbows, wrists, hands, iliac spine, hips, knees, ankles and feet on both sides; head, neck base, back.

By interpolating the points between each other, it is possible to gain information about posture and an analysis of the parameters characterizing the movement of the body. The movement sensors, and for example the accelerometers, are apparatuses which detect and send data concerning the acceleration on three axes; they function as receiving nodes, so that the information in the above described modes, analysis, interaction and chronology, are allowed to be processed.

They allow more precise and punctual detections on the position and movements, detecting the inertia of a mass when this one is subjected to an acceleration. Such sensors are applied to the anatomic portions object of evaluation and of possible rehabilitative intervention.

### Example 1

In the study of the upper limb movement, the sensors are applied at the following anatomical landmarks on both limbs:
- at the base of the wrist, between radius and ulna, with the forearm pronated;
- on the lateral epicondyle of the radio;
- at the humeral head, on the belly of the deltoid muscle (approximately 2 cm under the acromion);
- at the acromioclavicular articulation.

The movements and the parameters recorded are the following:
- flexo-extension of the wrist
- adduction-abduction of the wrist
- prono-supination of the forearm
- flexo-extension of the elbow
- flexo-extension of the back
- adduction-abduction of the back
- intra-extra rotation of the back
end each possible combination thereof.

Moreover, another sensor is positioned on the spinous process of the seventh cervical vertebra. Concerning the intrinsic movements of the hand, the sensor is represented by an interactive glove as for example a data glove, for recording the parameters as for example and not limited to:
- flexo-extension of the fingers
- flexo-extension of the phalanx
- flexo-extension of the middle phalanx
- adduction-abduction of the fingers
- thumb opposition to fingers
and each possible combination thereof, comprising the singularization of fingers.

### Example 2

In the study of the lower limb movement, the sensors are applied at the following anatomical landmarks on both limbs, as for example and not limited to:
- at anterior superior iliac spines level (SIAS)
- at posterior superior iliac spines level (SIPS)
- at greater trochanter level
- on the head of the fibula
- on lateral malleolus
- at the base of the first and fifth metatarsus in order to record the prono-supination of the foot.

It is important to evaluate how (time, speed and load) the foot rests on the ground and detaches itself from the ground (flexo-extension of the metatarso-phalangeal articulations), and which can be evaluated by means of a platform or a mat sensible to the load or by an interactive socks. Moreover, another sensor is positioned at the spinous process of the fourth lumbar vertebra and of the fifth sacral vertebra.

Concerning the intrinsic movements of the ankle and foot, the sensor can be represented by an interactive socks for recording the parameters such for example and not limited to:
- prono-supination of the foot
- angular deviation of the rear-forefoot
- flexo-extension of the tibio-tarsal articulation and of the tibio-tarsal articulation on the metatarso-phalangeal one
- flexo-extension of the fingers
- flexo-extension of the phalanxes
- flexo-extension of the middle phalanxes

For the aims of this method, all the devices/sensors which are able to quantify and measure the movement can be considered valid. In order to start the procedure of rehabilitative evaluation/training, sensors needed for the access to the selected virtual setting are applied to the subject.

The method collects all parameters detected by the sensors applied; among which, for example, concerning the lower limb and the balance:
- pressure and load force. The subject which uses the equipment could tend to shift his body weight more on one limb then on the other one. For this reason, it is important to understand when this happens and if the subject is able to balance the load. This parameter is recorded by using a sensor which detects the pressure.
- Movement performance speed. This parameter allows to record the performance speed of the movement in the various body areas and in various movement sequences and to compare the collected data with knowledge base made up of detections coming from national and/or international subjects. This allows to individuate the most difficult actions for the subject. This parameter is detected by applying sensors at the various articulations.

- Movement range. The subject usually experiences a reduced movement range of some articulations, because of the little use, a trauma or rigidity. It is important to monitor them and, when it is the case, to try to recover the lost articulation, substantially to do correct and functional movements. Concerning the lower limb for example, the following articulations are taken in consideration: the foot and the articulations of the fingers for the flexo-extension movement, the ankle, above all for the movement of plantar and dorsal flexion, the knee for the movement of flexion and extension, and the ankle, for the movement of flexion, extension, adduction and abduction. This parameter is recorded by means of sensors, arranged on the articulation examined.
- Foot speed, detachment and rest on the ground while walking. There are subjects in whom, while walking, the resting phase on one limb tends to be reduced and so the controlateral foot "advancing" speeds up; moreover the subject has usually muscle alterations and difficulties in controlling the various articulations (hip, knee, ankle and foot). All together these aspects lead to an incorrect foot rest and detachment from the ground. These parameters are recorded by sensors arranged at the various articulations and by a platform provided with pressure sensors or by the interactive sock.
- Step length. In order that the walking pattern is correct and fluid, it is needed that the two semi-steps, by means of the right and left lower limb, are approximately the same length. Moreover, it is important that each semi-step is long enough to allow the foot moving to go beyond the one resting on the ground. In the walking pattern of a subject suffering from any neuromotor dysfunction, these two parameters are not usually observed; for example, the semi-step performed by a limb is shorter than the controlateral one. These parameters are detected by using a motion controller and sensors positioned at the various articulations.
- Barycentre modifications. The barycentre shifting, both in antero-posterior and latero-lateral direction, are recorded by means of a platform with sensors or a mat detecting the pressure while the subject goes on it, and/or by position sensors.

Concerning the upper limb:
- Pressure or grasping force and hand releasing capacity. By means of this parameter, it is possible to evaluate the capacity of a subject to control the grasping movement, i.e. the hand closing around an object, and the releasing movement. This parameter is evaluated by using a virtual glove able to measure the pressure exerted by the fingers of the hand on an object, to detect the movement speed of flexion and extension of the fingers, of adduction and abduction of the fingers, of opposition of the thumb to the fingers of the hand, and to record these data by sending them to a central database. It is also possible to evaluate and study how a hand gets ready to receive a specific object and to make the suitable corrections in subjects suffering from specific difficulties.
- Movement performance speed. It is here evaluated the performance speed of the movements of the wrist, back and elbow articulations. This parameter is important because it allows to compare the performance speed of the two limbs in performing the same action in order to individuate the most difficult and demanding ones. The data concerning the speed are extrapolated by sensors arranged at the articulations as above described with reference to the equipment.

- Movement range. The subject usually experiences a reduced movement range of some articulations, because of the little use, a trauma or rigidity. It is important to monitor them and, when it is the case, to try to recover the lost articulation, substantially to do correct and functional movements. Concerning the upper limb, the reduction of articulation ROM can cause important functional limitations and motor compensations with consequent postural defects. For this reason, by using sensors, it is to evaluate the joint amplitude angles of the back in the flexion, extension, abduction, adduction and horizontal flexion movements, of the elbow in the flexion and extension movement, of the forearm concerning the prono-supination and of the wrist in the flexo-extension and adduction and abduction movements with the forearm in neutral position, in addition to the intrinsic movement of the hand.
- Approaching behavior to an object. It is examined the reaching capacity of the subject, i.e. how he reaches and takes an object lying before him. This action characterizes strongly the subject suffering from motor difficulties as the used motor pattern is usually rich in variable compensations different from subject to subject. This parameter is evaluated by using the data extrapolated by the sensors arranged at the back, elbow and wrist so that in the processing step, a movement model is provided by means of which it is possible to reconstruct the approaching behavior of each subject; moreover, the interactive glove will be used to analyze and evaluate the behavior of hand and fingers in approaching an object.

The adjustment occurs the first time the subject uses the equipment; it is needed in fact that this one records all the anthropometric parameters, as for example weight, height, prevailing side, and the not sensible personal ones, as for example gender, age, motor difficulties; in addition the subject is asked to express the pain level perceived through a validated evaluation; at the end of the action, this datum is compared to verify if the experience led to pain or not. If the subject made the task correctly and with no compensations. The user subject or the supervisor input these data in order to allow both a correct use of the equipment and the recording of a personal ID which can be used in the following for a population study. At the first use of the virtual setting/play-motor experience, it is needed an adjustment/balance of the setting features on the basis of the functional conditions of the subject: the subject is asked to do simple but specific movements in order to collect measures considered as primary pattern. This procedure is also needed to adapt the proposal to the motor difficulty level of the user subject so that the requested task is the most possible targeted to his functional needs, in addition to allow a most gradual possible access/accessibility to the equipment.

The play-motor experience occurs in a virtual setting, which is provided ad hoc, snappy, involving and stimulating for the user subject as well as original, exclusive and tailored-made for him; he can choose different options also in function of his desires and hobbies. Each setting/play has a specific goal and finalized functional requests the subject has to respond to during the play activity. The subject is led to carry out studied and targeted patterns (in relation to his own profile and his own motor difficulty situation), by activating specific motor units in order to end successfully the rehabilitative training set up in the virtual setting. Each virtual setting is structured on more difficulty levels to make the task always more rigid and to recall more attention of the involved subject; there is no motor learning in fact, if there is no attention on the requested task. For example, the subject can be asked to walk on a virtual path where the floor is made up of stones and ice panels with ever more thinner layers; the subject has to pay attention not to break the ice, otherwise he would fall in a frozen lake and so he has to pace the load on both the limbs. At the end of the activity, the user subject is asked to express again the pain level perceived; also this datum is collected and analyzed and allows the supervisor to make possible modifications to the rehabilitative training of the subject.

During the activity, the data concerning the motor performance of the user subject are recorded so that the supervisor can evaluate the rehabilitative training and make corrections of the motor program errors. The information concerning the various parameters indicated are collected by the sensors during the play-motor activity in the virtual setting, and are different according to the motor difficulty of the subject.

Such data are then filed in a local database and, in the following, if the subject allows to send the data to the central database, they are sent by means of an internet connection. The method is provided with the collection and processing of the information collected by the sensors by means of a dedicated software which analyses the acquired data at the end of the action and compares them with the primary pattern and with the data concerning every activity carried out by the subject for that precise setting. This allows to verify, upon intelligible presentation, the set up training development, both in the condition that the subject is carrying it out in "assisted mode" and in "independent mode", and to make suitable corrections. If the subject is carrying out training and is using the setting in "assisted mode", the supervisor will control the presentation of the data processing and will make the suitable corrections of the functional parameters of the equipment so that the subject can provide his best performance in order to reach the activity aim. Such indications, if the subject is using the equipment in independent mode, are provided from the same setting.

## Claims

1. Method for neuromotor re-education which uses virtual and interactive setting systems which allow the subject to carry out rehabilitative training by means of play activities, **characterized by** the following steps:
- adjustment/balancing of the setting features according to the subject functional conditions;
- detections by means of sensors and collection of patient parameters;
- analysis of the collected data;
- interaction of the collected data;
- chronologizing of the collected data;
- data filing in a local database;
- data filing in a general database;
- data processing for defining the rehabilitative program.

2. Method according to claim 1, **characterized in that** it can be carried out according to two modes: the assisted and independent one.

3. Method according to claim 2, **characterized in that** said assisted mode is made up of two fundamental parties which are the patient and the supervisor and **in that** the independent mode is made up of only the patient who carries out the activity.

4. Method according to claim 2 or 3, **characterized in that** it allows the supervisor to get an updated view of the subject performance, and to interact with him by means of a message system in order to suggest/correct/propose the next activities or the suitable settings.

5. Method according to any one of claims 1 to 4, **characterized by** the integration of an algorithm for screening and analyzing data and to provide a determinist method for the evaluation of the corrective actions.

6. Method according to any one of the preceding claims, **characterized in that** it is possible to self-regulate the rehabilitative training management on the basis of the patient's answers, thus allowing to adapt the activities in relation to the functional needs.

7. Method according to any one of the preceding claims, **characterized in that** the virtual setting used are original, exclusive and tailored-made, and individuated and provided as well for every single motor disorder in order to allow to summon up selective muscle areas and the activation of specific motor units and specific osteoarticular districts.

8. Apparatus to carry out the method according to any one of claims 1 to 7, **characterized by** the provision of a motion controller, a posturometric platform and a plurality of sensors.

9. Apparatus according to claim 8, **characterized in that** said posturometric platform is able to measure the barycentre, the mass and body weight of the user exactly.

10. Apparatus according to claim 8, **characterized in that** said sensors are interactive socks applied to the anatomical areas object of interest and of possible rehabilitative intervention, and detect and send the data relative to the acceleration on three axes.

11. Processing computer code for implementing the method according to any one of claims 1 to 7, **characterized in that** it manages both the reproduction of specific virtual settings and the processing of the specific collected data.

12. Processing code according to claim 11, **characterized in that** it displays the data collected in real time by the platform, both numerically and graphically.

13. Use of the present method and apparatus as well as of the processing computer code according to the preceding claims, for providing a national and international database, whose consultation allows to analyze the epidemiologic phenomena statistically.
